# EUROPÄISCHE PATENTSCHRIFT

(11) **EP 0 555 699 B1**
(45) Veröffentlichungstag und Bekanntmachung des Hinweises auf die Patenterteilung: **07.01.1998**
(21) Anmeldenummer: 93101291.8
(22) Anmeldetag: 28.01.1993
(51) Int. Cl.: C09B 43/44, C09B 43/124, C07C 309/51, C07C 309/52, C07C 271/28, C09B 29/30

(54) **Verfahren zur Herstelung von Aminourethanen**
Process for the preparation of aminourethanes
Procédé de préparation des urethanes amino

(30) Priorität: 10.02.1992 DE 4203795
(43) Veröffentlichungstag der Anmeldung: 18.08.1993
(73) Patentinhaber: BAYER AG, 51368 Leverkusen (DE)
(72) Erfinder: Jäger, Horst, Dr., W-5090 Leverkusen 1 (DE); Henk, Hermann, Dr., W-5000 Köln 80 (DE)

(56) Entgegenhaltungen:
- EP-A- 0 402 318
- EP-A- 0 474 039
- CH-A- 591 544
- FR-A- 2 286 175
- US-A- 4 080 322
- DATABASE WPI Week 8129, Derwent Publications Ltd., London, GB; AN 81-52264D[29] & JP-A-56 062 821 (HITACHI CHEMICAL KK) 29. Mai 1981 & JP-A-56 062 821 (...)

## Beschreibung

Die Erfindung betrifft ein neues Verfahren zur Herstellung von Verbindungen, die mit einer Urethan- und einer Aminogruppe substituiert sind.

Aus der EP-A-402 318 sind Aminophenole bekannt, die eine Urethangruppierung aufweisen. Andere Verbindungen als Phenole werden hierbei nicht angegeben.

Aus CH-A-591 544 sowie aus US-A-4 080 322 sind Urethane bekannt, die gemäß ihrer generischen Formel (I) auch Aminourethane umfassen. Die Herstellung der bevorzugten Urethane der Formel (I) erfolgt durch Umsetzung von Diaminoverbindungen mit Halogenameisensäureestern, wobei Bis-urethan-Verbindungen entstehen. Gemäß DE-A 2 528 370 werden spezielle Aminourethane durch Umsetzung von den entsprechenden Diaminen mit Chlorameisensäureestern oder durch Kupplung von Urethangruppen-haltigen Diazokomponenten auf Aminogruppen-haltige Kupplungskomponenten hergestellt.

Gemäß Abstract von JP-A-56 06 28 21 werden Polymere unter Verwendung von Aminourethanen als Ausgangsprodukt hergestellt.

Die vorliegende Erfindung betrifft ein Verfahren zur Herstellung von Verbindungen der Formel 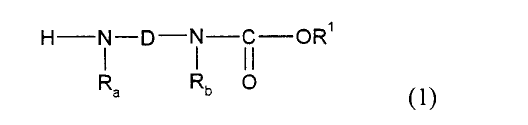 worin
- Rₐ,R_{b}: unabhänig voneinander Wasserstoff oder gegebenenfalls substitutiertes Alkyl,
- R¹: gegebenenfalls substituiertes Alkyl, Aralkyl, Aryl oder Cycloalkyl und
- D: einen zweiwertigen aromatischen Rest bedeuten,
ausgenommen derartige Verbindungen, bei denen D = OH-substituiertes Phenyl sowie ausgenommen die in der DE-A-40 27 970 angegebenen Verbindungen der Formel (I) 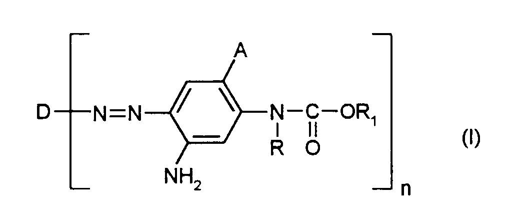 worin
- D: für den Rest einer Diazokomponente steht,
- A: für Wasserstoff, Alkyl, Alkoxy, Halogen, Carboxy oder Sulfo steht,
- R: für Wasserstoff oder Alkyl steht,
- R₁: für Alkyl, Aralkyl, Aryl oder Cycloalkyl steht, und
- n: für 1 oder 2 steht,
insbesondere sind derartige Verbindungen ausgenommen, bei denen eine Azogruppe in Parastellung zur Gruppierung R¹-O-CO-NR_{b}- steht, das dadurch gekennzeichnet ist, daß man Verbindungen der Formel 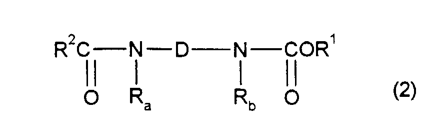 worin
- Rₐ, R_{b}, R¹ und D: die oben angegebene Bedeutung haben,
- R²: für Wasserstoff, Alkyl, Alkenyl, Aralkyl, Phenyl oder Carboxy steht,
im sauren Medium verseift.

In einer bevorzugten Ausführungsform steht
- D: vorzugsweise für einen gegebenenfalls substituierten Rest der Benzol-, Naphthalin-, Diphenyl-, Stilben-, Benzolazobenzol-, Benzolazonaphthalin-, Naphthalinazonaphthalin- oder heterocyclischen Reihe,
- Rₐ, R_{b}: vorzugsweise für Wasserstoff oder gegebenenfalls substituiertes C₁-C₄-Alkyl,
- R¹: vorzugsweise für gegebenenfalls substituiertes C₁-C₄-Alkyl, gegebenenfalls im Phenylteil substituiertes Phenyl(C₁-C₂)alkyl, gegebenenfalls substituiertes Phenyl oder gegebenenfalls substituiertes C₅-C₆-Cycloalkyl.

Beispiele für geeignete Substituenten des Restes D sind: C₁-C₄-Alkyl, C₁-C₄-Alkoxy, wobei diese Alkyl- oder Alkoxyreste beispielsweise durch OH, -OCH₃, -COOH, -OSO₃H, -SO₃H substituiert sein können; -SO₃H, -COOH, Halogenatome wie F, Cl oder Br; NH₂, OH, NO₂, Phenylamino, wobei der Phenylrest beispielsweise durch CH₃, -OCH₃, -SO₃H substituiert sein kann; Phenyl- und Naphthylazo, wobei diese Reste insbesondere durch -SO₃H substituiert sein können.

Ein bevorzugter Substituent des Restes D ist die Sulfogruppe.

Beispiele für gegebenenfalls substituiertes C₁-C₄-Alkyl in der Definition von Rₐ und R_{b} sind Methyl, Ethyl, n-Propyl, iso-Propyl, n-Butyl, s-Butyl, i-Butyl oder t-Butyl, wobei als Substituenten jeweils beispielsweise in Frage kommen: OH, -OCH₃, -COOH, -OSO₃H oder -SO₃H.

Beispiele für Reste Rₐ und R_{b} sind -CH₃, -C₂H₅, -C₂H₅OH, -CH₂-CH₂COOH, -CH₂CH₂SO₃H.

Beispiele für Reste R¹ sind -CH₃, -C₂H₅, -CH(CH₃)₂, -CH₂CH₂CH₂CH₃,

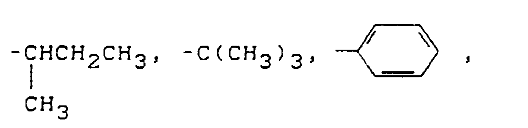

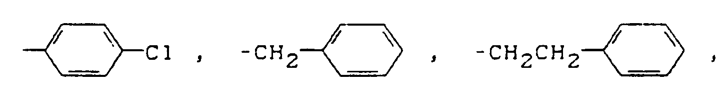

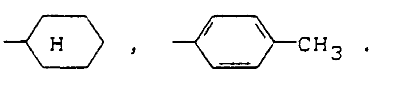

Bevorzugte Substituenten für R¹ sind: Cl, OCH₃, CH₃.

Bevorzugte Verbindungen im Rahmen der Formel (1) sind solche, in denen
D für 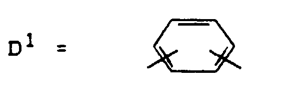 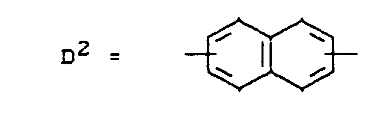 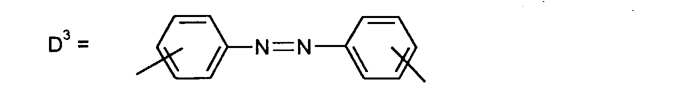 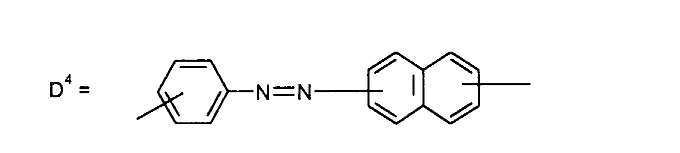 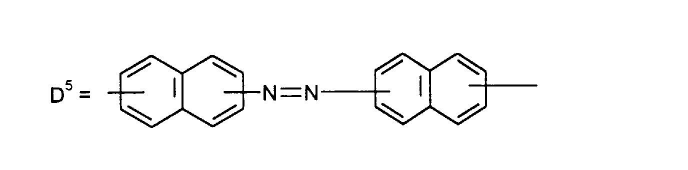 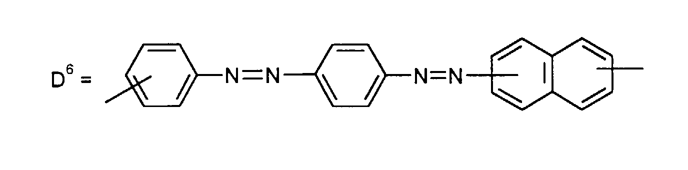 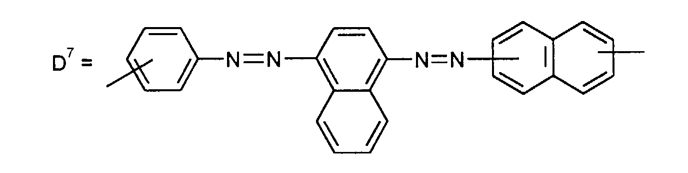 steht
worin die Benzol- bzw. Naphthalinreste in der Bedeutung D¹ bis D⁷ beispielhaft die für D genannten Substituenten enthalten können, insbesondere Sulfogruppen.

Weiterhin bevorzugt sind Verbindungen der Formel (1) und solche mit der bevorzugten Bedeutung für D, in denen R¹ für Methyl oder Ethyl steht und Rₐ und R_{b} für Wasserstoff, Methyl oder Ethyl steht.

Die für R² genannten Reste können substituiert sein, insbesondere mit OH, SO₃H, Cl, COOH, OSO₃H. Beispiele für Reste R² sind:
-CH₃, -C₂H₅, -C₃H₇, iso-C₃H₇, -CH₂Cl, -CH₂OH, -CH₂OSO₃H, -CH₂SO₃H, -CH=CHCOOH,

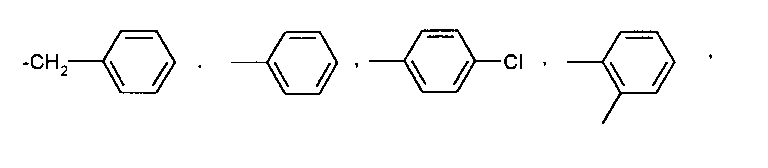

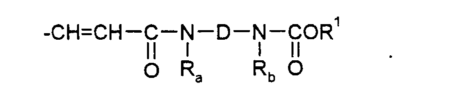

Die bveorzugte Bedeutung von R² ist CH₃.

Die Verseifung der Verbindungen (2) zu den Verbindungen (1) geschieht in einem Gemisch aus Wasser und einem organischen Lösungsmittel, vorzugsweise in Wasser, durch Erhitzen auf 60 bis 110°C, insbesondere 80 bis 90°C, in Gegenwart von Säure. Der Gehalt an Säure liegt bei 0,5 bis 3 Mol, vorzugsweise bei 1 bis 2 Mol Säure pro Liter Verseifungslösung. Als Säure verwendet man insbesondere Salz- oder Schwefelsäure. Als organische Lösungsmittel verwendet man insbesondere solche, die mit Wasser mischbar sind, wie Alkohole, Ether, Ketone und andere. Beispiele sind: Methanol, Ethanol, Isopropanol, n-Butanol, Aceton, Methylethylketon, Diethylketon, Ethylenglykol, Diethylenglykol, Ethylenglykolmonomethylether, Ethylenglykoldimethylether, Dioxan, Sulfolan.

Die Verbindungen der Formel (2) erhält man beispielsweise durch Acylierung von Verbindungen der Formel 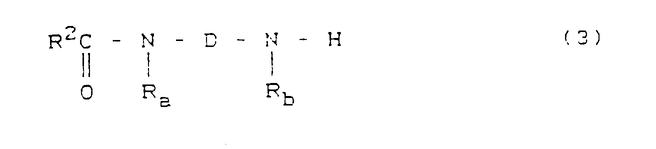 worin
- R², Rₐ, R_{b} und D: die angegebene Bedeutung haben,
mit Chlorameisensäureestern der Formel

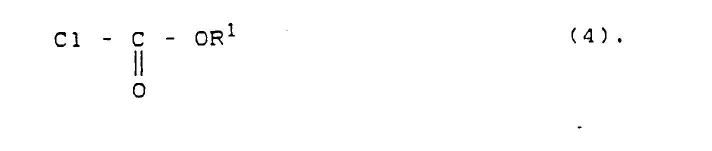

Die Acylierung der Verbindungen (3) mit den ameisensäureestern (4) wird vorzugsweise in Wasser bei 0 bis 30°C, insbesondere 5 bis 15°C, und bei pH 3 bis 8, insbesondere 5 bis 7, ausgeführt, wobei die bei der Acylierung freiwerdende Salzsäure durch Zusatz von Alkali- oder Erdalkalihydroxiden, -carbonaten, -hydrogencarbonaten oder -phosphaten neutralisiert wird.

Verbindungen der Formel (2) erhält man weiterhin für den Fall, daß D die bevorzugte Bedeutung von D³ bis D⁷ hat, dadurch, daß man Diazokomponenten und Kupplungskomponenten verwendet, von denen die eine den Rest 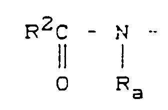 und die andere den Rest 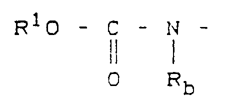 enthält.

Beispiele für geeignete Amine (3) mit D = D¹ sind:
1-Amino-3-acetylamino-benzol-6-sulfonsäure
1-Amino-4-acetylamino-benzol-6-sulfonsäure
1-Amino-4-acetylamino-3-methoxy-benzol-5-sulfonsäure
1-Amino-3-acetylamino-benzol
1-Amino-4-acetylamino-benzol
1-Amino-3-acetylamino-benzol-6-carbonsäure
1-Amino-4-acetylamino-benzol-6-carbonsäure
D = D²:
2-Acetylamino-5-amino-naphthalin-4,8-disulfonsäure
D = D³:
1-Amino-3-methyl-benzol-<4 azo 1> 2-sulfo-4-acetylaminobenzol
1-Amino-3-methyl-benzol-<4 azo 1> 2,5-disulfo-4-acetylamino-benzol
D = D⁴:
1-Amino-7-sulfo-naphthalin-<4 azo 1> 2-sulfo-4-acetylamino-benzol
1-Amino-6-sulfo-naphthalin-<4 azo 1> 2-sulfo-4-acetylamino-benzol
1-Amino-3-methyl-benzol-<4 azo 2> 4,8-disulfo-6-acetylamino-naphthalin
D = D⁵:
1-Amino-7-sulfo-naphthalin-<4 azo 1> 4-acetylamino-7-sulfo-naphthalin

Beispiele für Chlorameisensäureester der Formel (4) sind:
Chlorameisensäuremethylester,
Chlorameisensäureethylester,
Chlorameisensäure-iso-propylester,
Chlorameisensäure-n-propylester,
Chlorameisensäure-benzylester.

Beispiele für Diazo- und Kupplungskomponenten, durch deren Vereinigung Verbindungen der Formel (2) entstehen, in denen D die bevorzugte Bedeutung von D³ bis D⁷ hat, sind folgende:

Diazokomponenten mit einem Rest 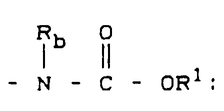
1-Amino-3-ethoxycarbonylamino-benzol-6-sulfonsäure
1-Amino-4-ethoxycarbonylamino-benzol-2-sulfonsäure
1-Amino-3-ethoxycarbonylamino-benzol
1-Amino-4-ethoxycarbonylamino-benzol-2-carbonsäure
1-Amino-3-ethoxycarbonylamino-benzol-6-carbonsäure

Diazokomponenten mit einem Rest 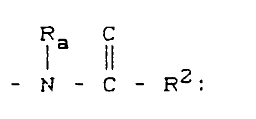
1-Amino-4-acetylamino-benzol
1-Amino-3-acetylamino-benzol
1-Amino-2-acetylamino-benzol
1-Amino-4-acetylamino-benzol-2-sulfonsäure
1-Amino-5-acetylamino-benzol-2-sulfonsäure
1-Amino-2-acetylamino-benzol-5-sulfonsäure
1-Amino-4-acetylamino-benzol-3-sulfonsäure
1-Amino-4-acetylamino-3-methoxy-benzol-5-sulfonsäure
1-Amino-4-acetylamino-benzol-2,5-disulfonsäure
1-Amino-4-acetylamino-benzol-2-carbonsäure
1-Amino-5-acetylamino-benzol-2-carbonsäure
1-Amino-4-acetylamino-2,5-dimethoxy-benzol.

Kupplungskomponenten mit einem Rest 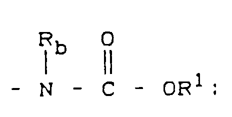
2-Ethoxycarbonylamino-5-hydroxy-naphthalin-7-sulfonsäure (hergestellt nach den Angaben des Beispiels A der europäischen Patentschrift 406 629)
3-Ethoxycarbonylamino-5-hydroxy-naphthalin-7-sulfonsäure
3-Ethoxycarbonylamino-5-hydroxy-naphthalin-2,7-disulfonsäure
8-Ethoxycarbonylamino-1-hydroxy-naphthalin-3,6-disulfonsäure
8-Ethoxycarbonylamino-1-hydroxy-naphthalin-3,5-disulfonsäure

Kupplungskomponenten mit einem Rest 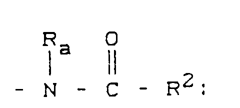
2-Acetylamino-5-hydroxy-naphthalin-3-sulfonsäure
3-Acetylamino-5-hydroxy-naphthalin-3-sulfonsäure
8-Acetylamino-1-hydroxy-naphthalin-3,6-disulfonsäure
8-Acetylamino-1-hydroxy-naphthalin-3,5-disulfonsäure.

Die Formeln der wasserlöslichen Verbindungen in der Beschreibung und in den Beispielen sind die der freien Säuren. Isoliert und angewandt werden die Stoffe im allgemeinen in Form ihrer Alkalisalze, insbesondere der Lithium-, Natrium- oder Kaliumsalze.

Die Verbindungen der Formel (1) sind wertvolle Zwischenprodukte zur Herstellung von Reaktiv- und Substantivfarbstoffen.

### Beispiel 1

23,0 g 1-Amino-3-acetylamino-benzol-6-sulfonsäure werden in 200 ml Eiswasser neutral gelöst. Man tropft in einer Stunde 11 g Chlorameisensäureethylester ein und hält dabei durch gleichzeitige Zugabe von verdünnter Sodalösung einen pH von 5 bis 6 in. Wenn eine Probe (Diazotierung und Kupplung auf H-Säure im sodaalkalischen Bereich) vollständige Acylierung anzeigt, setzt man soviel konzentrierte Salzsäure ein, daß eine ca. 2n-Salzsäure vorliegt und erwärmt auf 80 bis 90°C bis die Acetylgruppe abgespalten ist. Das Reaktionsprodukt scheidet sich als inneres Salz (betain) ab. Es wird abgesaugt, die anhaftende Säure durch Waschen mit Wasser entfernt und der Rückstand getrocknet. Es resultiert eine farblose Substanz mit der Formel

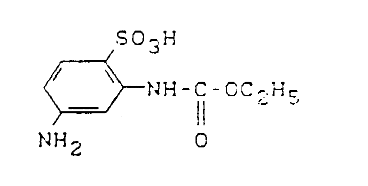

Wenn man nach den Angaben von Beispiel 1 verfährt und die in Spalte 1 der Tabelle 1 aufgeführten Amine verwendet, so erhält man die in Spalte 2 genannten Amine.

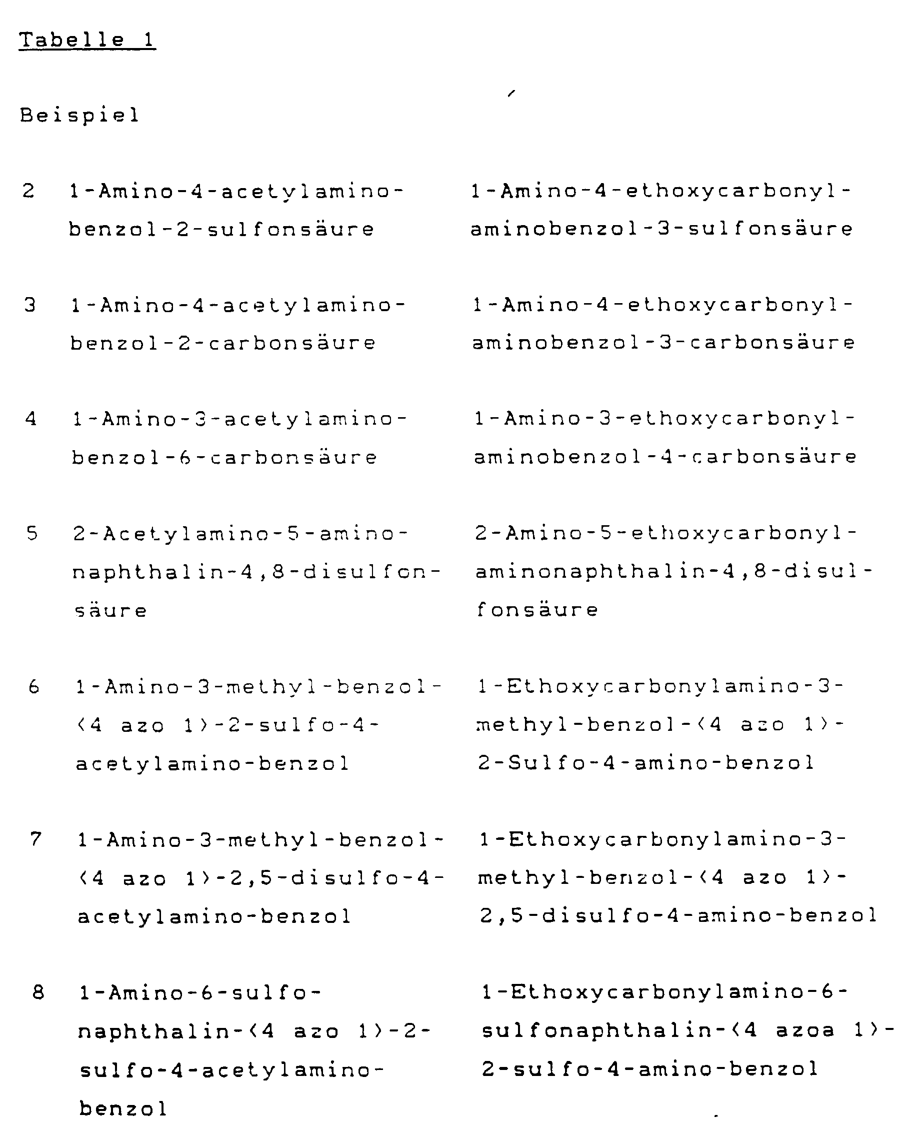

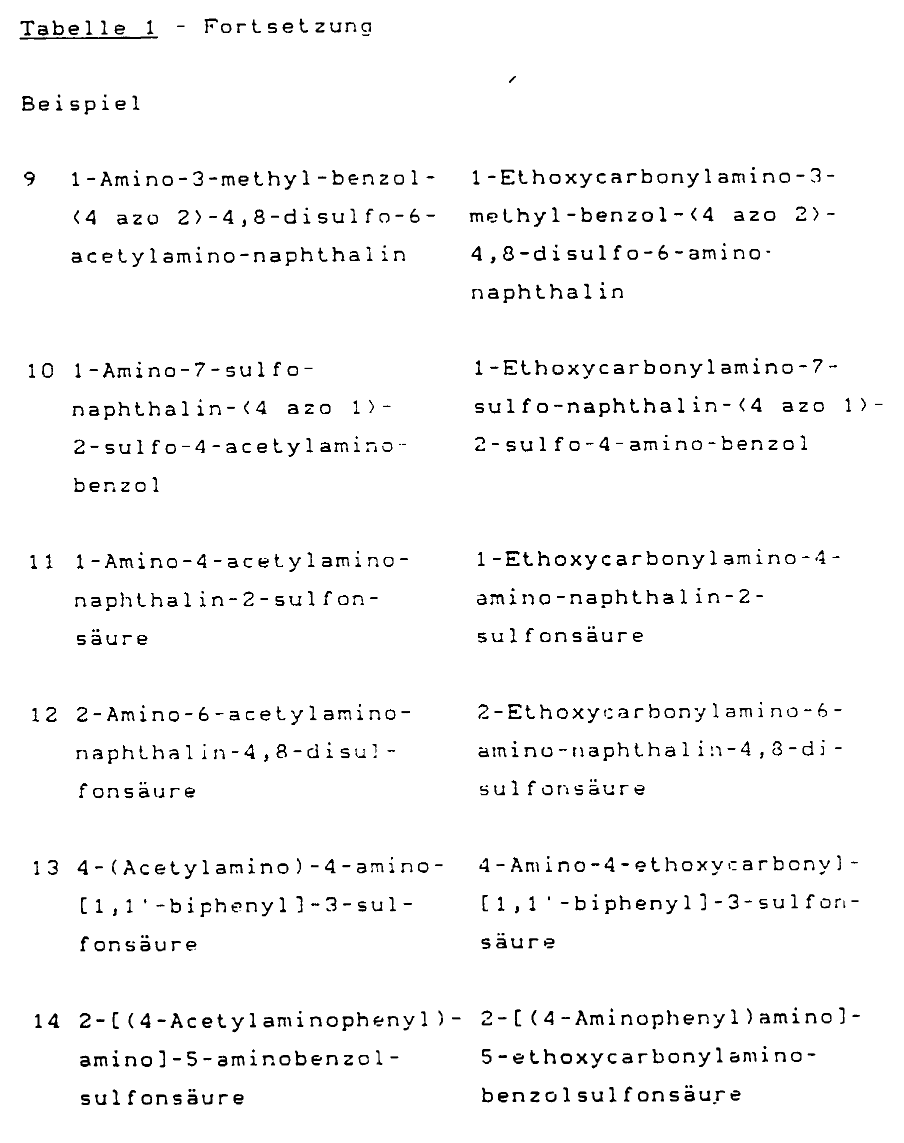

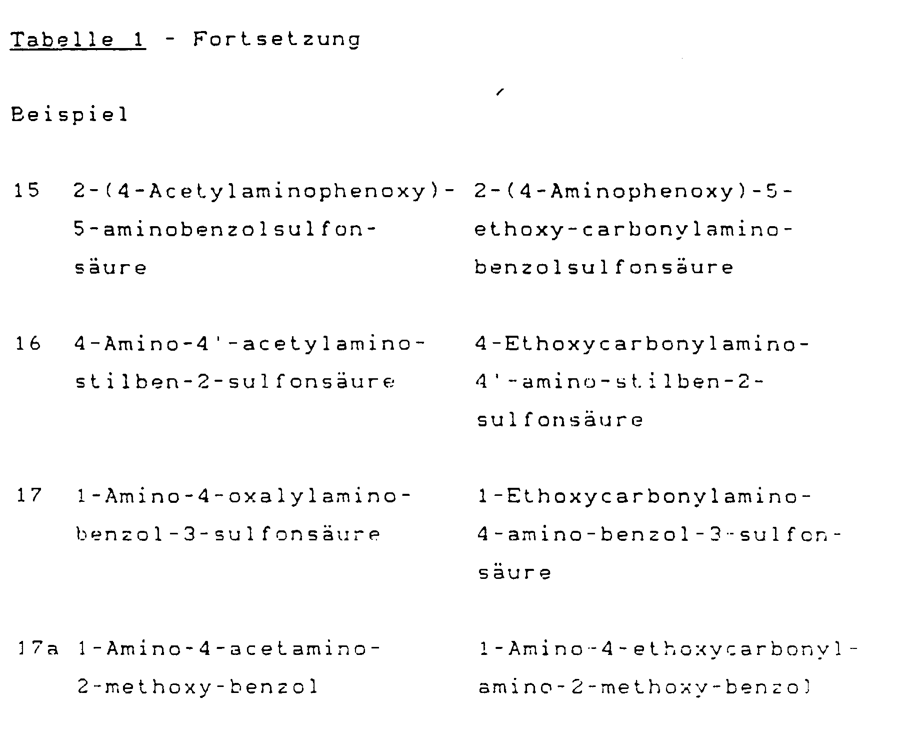

### Beispiel 18

31,1 g 1-Hydroxy-6-ethoxycarbonylaminonaphthalin-3-sulfonsäure (hergestellt nach den Angaben von Beispiel A der EP-A-406 629), die als neutrales Natriumsalz in 300 ml Wasser gelöst sind, werden bei 0 bis 5°C mit der aus 26,0 g 1-Amino-4-oxalylamino-benzol-3-sulfonsäure in üblicher Weise erhaltenen Diazoverbindung im Laufe einer Stunde versetzt, wobei man gleichzeitig Natriumhydrogencarbonat einstreut, um den pH während der Kupplung zwischen 5,0 und 5,5 zu halten. Zur Vervollständigung der Kupplung rührt man 2 Stunden nach. Der Farbstoff ist zum größten Teil ausgefallen. Zur Verseifung setzt man dann soviel konzentrierte Salzsäure zu, daß eine ca. 2n-Salzsäure vorliegt, und erwärmt solange auf 85° bis das Dünnschichtchromatogramm das Ende der Verseifung anzeigt. Der Farbstoff wird warm abgesaugt. Die resultierende Paste löst sich neutral in Wasser mit roter Farbe.

Der Farbstoff entspricht folgender Formel:

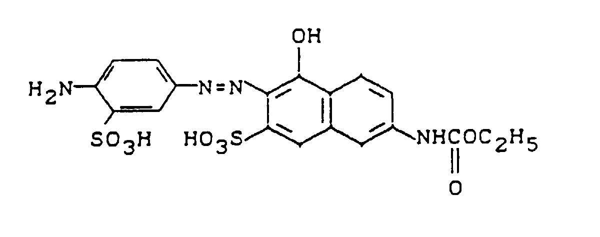

Weitere Aminoazofarbstoffe erhält man nach den Angaben von Beispiel 17, wenn man Diazokomponenten und Kupplungskomponenten gemäß Tabelle 2 verwendet.

**Tabelle 2**

| Beispiel | Diazokomponente | Kupplungskomponente |
|---|---|---|
| 19 | 1-Amino-4-acetyl amino-benzol-2-sulfonsäure | 1-Hydroxy-6-ethoxy-carbonyl-amino-naphthalin-3-sulfonsäure |
| 20 | 1-Amino-4-acetyl-amino-benzol-2,5-disulfonsäure | " |
| 21 | 1-Amino-5-acetyl-amino-benzol-2-sulfonsäure | " |
| 22 | 1-Amino-4-acetyl-amino-naphthalin-7-sulfonsäure | " |
| 23 | 1-Amino-2-acetyl-amino-benzol-x-sulfonsäure | " |
| 24 | 1-Amino-4-acetyl-amino-benzol-3-sulfonsäure | 1-Hydroxy-7-ethoxy-carbonyl-amino-naphthalin-3-sulfonsäure |
| 25 | 1-Amino-4-acetyl-amino-benzol-2-sulfonsäure | " |

### Beispiel 26

26,0 g 1-Amino-4-ethoxycarbonylamino-benzol-2-sulfonsäure (erhalten nach den Angaben von Beispiel 17) werden in üblicher Weise indirekt diazotiert. Die Diazotierung gibt man bei 0° bis 5° in einer Stunde zu einer Lösung von 28,1 g 2-Acetylamino-5-hydroxy-naphthalin-7-sulfonsäure, wobei man durch gleichzeitiges Einstreuen von Calciumcarbonat einen pH von 5 bis 6 einhält. Nach beendeter Kupplung setzt man soviel konzentrierte Salzsäure zu, daß eine ca. 2n-Salzsäure vorliegt. Man erwärmt dann solange auf 85° bis das Dünnschichtchromatogramm das Ende der Verseifung der Acetylaminogruppe anzeigt. Der Farbstoff wird warm abgesaugt. Die Paste löst sich nach dem Neutralisieren mit roter Farbe in Wasser.

Der Farbstoff entspricht folgender Formel:

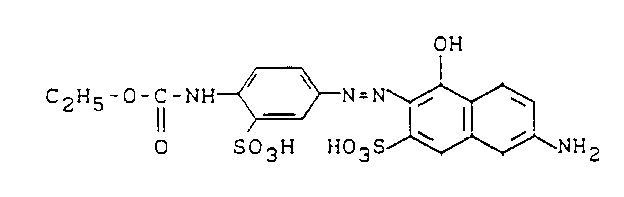

Weitere Aminoazofarbstoffe erhält man nach den Angaben von Beispiel 26, wenn man Diazokomponenten und Kupplungskomponenten gemäß Tabelle 3 verwendet.

**Tabelle 3**

| Beispiel | Diazokomponente | Kupplungskomponente |
|---|---|---|
| 27 | 1-Amino-4-ethoxy-carbonylamino-benzol-3-sulfonsäure (hergestellt nach den Angaben von Beispiel 2) | 2-Acetylamino-5-hydroxy-naphthalin-7-sulfonsäure |
| 28 | " | 3-Acetylamino-5-hydroxy-naphthalin-7-sulfonsäure |
| 29 | " | 8-Acetylamino-1-hydroxy-naphthalin-3,6-disulfonsäure |
| 30 | 1-Ethoxycarbonyl-amino-3-methyl-benzol-<4 azo 1>-2,5-disulfo-4-amino-benzol (hergestellt nach den Angaben von Beispiel 7) | 2-Acetylamino-5-hydroxy-naphthalin-7-sulfonsäure |

## Patentansprüche

1. Verfahren zur Herstellung von Verbindungen der Formel 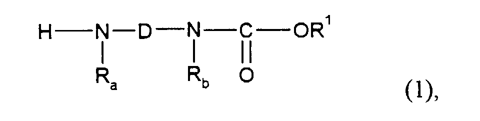 worin
Rₐ,R_{b} unabhänig voneinander Wasserstoff oder gegebenenfalls substitutiertes Alkyl,
R¹ gegebenenfalls substituiertes Alkyl, Aralkyl, Aryl oder Cycloalkyl und
D einen zweiwertigen aromatischen Rest bedeuten,
ausgenommen derartige Verbindungen, bei denen D = OH-substituiertes Phenyl sowie ausgenommen die in der DE-A-40 27 970 angegebenen Verbindungen der Formel (I) 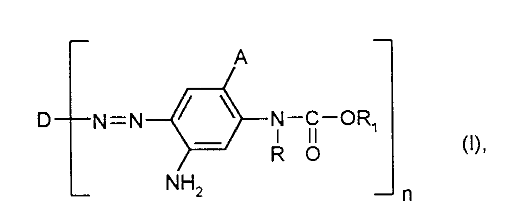 worin
D für den Rest einer Diazokomponente steht,
A für Wasserstoff, Alkyl, Alkoxy, Halogen, Carboxy oder Sulfo steht,
R für Wasserstoff oder Alkyl steht,
R₁ für Alkyl, Aralkyl, Aryl oder Cycloalkyl steht, und
n für 1 oder 2 steht,
dadurch gekennzeichnet ist, daß man Verbindungen der Formel (2) 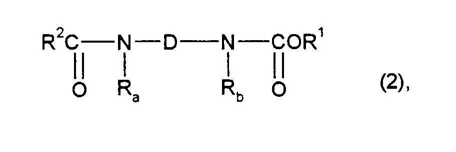 worin
Rₐ, R_{b}, R¹ und D die oben angegebene Bedeutung haben und
R² für Wasserstoff, Alkyl, Alkenyl, Aralkyl, Phenyl oder Carboxy steht,
im sauren Medium verseift.

2. Verfahren gemäß Anspruch 1, worin in Formel (I)
D für einen gegebenenfalls substituierten Rest der Benzol-, Naphthalin-, Diphenyl-, Stilben-, Benzolazobenzol-, Benzolazonapthalin-, Napthalinazonapthalin- oder heterocyclischen Reihe,
Rₐ, R_{b} für Wasserstoff oder gegebenenfalls substituiertes C₁-C₄-Alkyl,
R¹ für gegebenenfalls substituiertes C₁-C₄-Alkyl, gegebenenfalls im Phenylteil substituiertes Phenyl(C₁-C₂)alkyl, gegebenenfalls substituiertes Phenyl oder gegebenenfalls substituiertes C₅-C₆-Cycloalkyl steht.

3. Verfahren gemäß Anspruch 1, dadurch gekennzeichnet, daß in Formel (I) D substituiert ist durch C₁-C₄-Alkyl, C₁-C₄-Alkoxy, wobei diese Alkyl- oder Alkoxyreste durch OH, -OCH₃, -COOH, -OSO₃H, -SO₃H substituiert sein können; -SO₃H, -COOH, Halogenatome wie F, Cl oder Br; NH₂, OH, NO₂, Phenylamino, wobei der Phenylrest durch CH₃, -OCH₃, -SO₃H substituiert sein kann; Phenyl- und Naphthylazo, wobei diese Reste durch -SO₃H substituiert sein können.

4. Verfahren gemäß wenigstens einem der vorhergehenden Ansprüche, dadurch gekennzeichnet, daß in Formel (I) D steht für 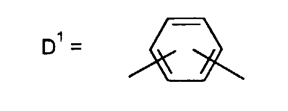 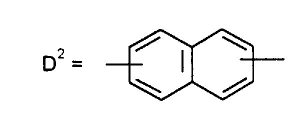 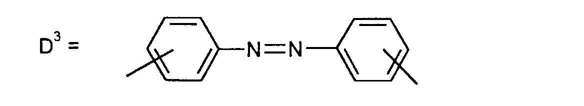 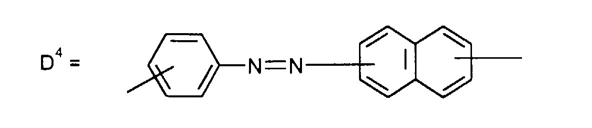 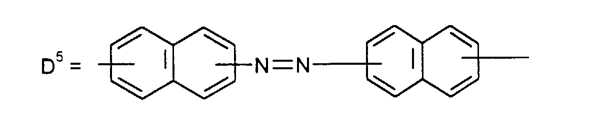 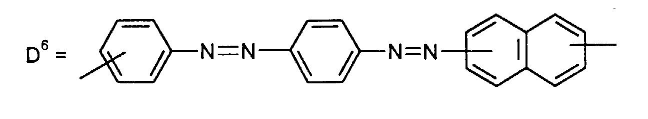 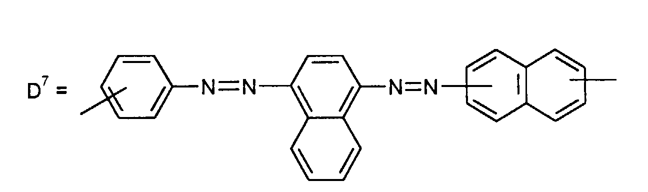 steht.

5. Verfahren gemäß Anspruch 1, dadurch gekennzeichnet, daß die Verseifung bei 60 bis 110° und in einem Gemisch aus Wasser und einem organischen Lösungsmittel oder in Wasser mit einem Gehalt von 0,5 bis 3,0 Mol Säure pro 1 durchgeführt wird.

6. Verfahren gemäß Anspruch 5, dadurch gekennzeichnet, daß man die Verseifung in Wasser ausführt.

## Claims

1. Process for the preparation of compounds of the formula 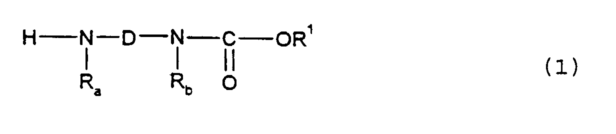 in which
Rₐ, R_{b} independently of one another, denote hydrogen or unsubstituted or substituted alkyl,
R¹ denotes substituted or unsubstituted alkyl, aralkyl, aryl or cycloalkyl and
D denotes a divalent aromatic radical,
with the exception of those compounds in which D is OH-substituted phenyl and with the exception of the compounds of the formula (I) mentioned in DE-A-4,027,970: 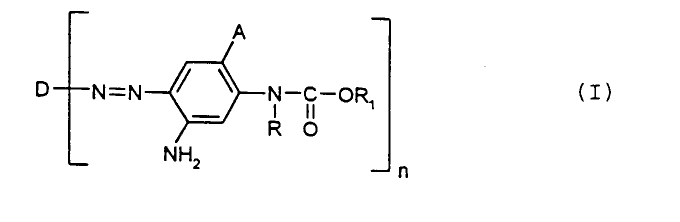 in which
D represents the radical of a diazo component,
A represents hydrogen, alkyl, alkoxy, halogen, carboxyl or sulpho,
R represents hydrogen or alkyl
R¹ represents alkyl, aralkyl, aryl or cycloalkyl, and
n represents 1 or 2, characterised in that compounds of the formula (2) 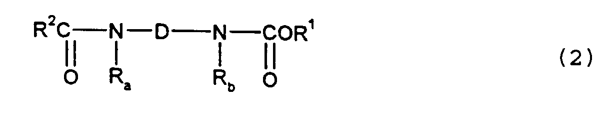
in which
Rₐ, R_{b}, R¹ and D have the abovementioned meaning, and
R² represents hydrogen, alkyl, alkenyl, aralkyl, phenyl or carboxyl,
are hydrolysed in an acidic medium.

2. Process according to Claim 1, in which in the formula (1)
D represents a substituted or unsubstituted radical of the benzene, naphthalene, biphenyl, stilbene, benzeneazobenzene, benzeneazonaphthalene, naphthaleneazonaphthalene or heterocyclic series,
Rₐ, R_{b} represent hydrogen or substituted or unsubstituted C₁-C₄-alkyl,
R¹ represents substituted or unsubstituted C₁-C₄-alkyl, phenyl(C₁-C₂)alkyl which is substituted or unsubstituted in the phenyl moiety, substituted or unsubstituted phenyl or substituted or unsubstituted C₅-C₆-cycloalkyl.

3. Process according to Claim 1, characterised in that D in the formula (1) is substituted by C₁-C₄-alkyl, C₁-C₄-alkoxy, it being possible for these alkyl or alkoxy radicals to be substituted by OH, -OCH₃, -COOH, -OSO₃H, -SO₃H; -SO₃H, -COOH, halogen atoms, such as F, Cl or Br; NH₂, OH, NO₂, phenylamino, it being possible for the phenyl radical to be substituted by CH₃, -OCH₃, -SO₃H; phenyl- and naphthylazo, it being possible for these radicals to be substituted by -SO₃H.

4. Process according to at least one of the preceding claims, characterised in that D in the formula (1) represents

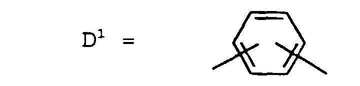

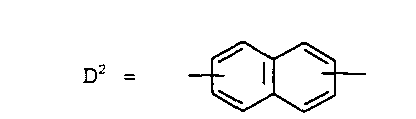

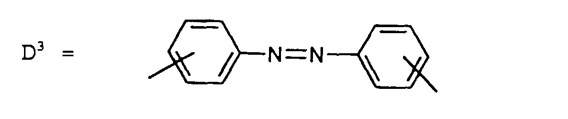

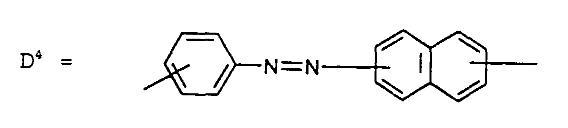

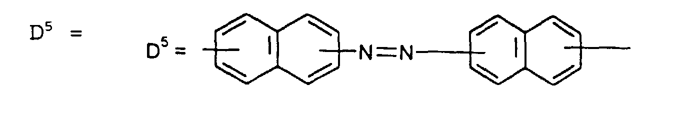

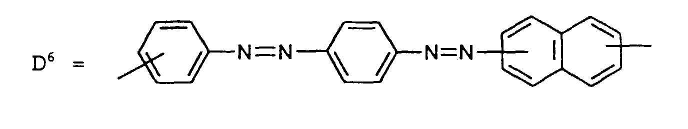

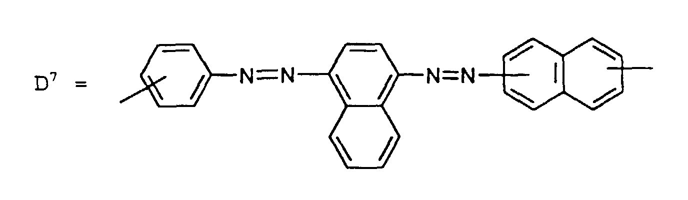

5. Process according to Claim 1, characterised in that the hydrolysis is carried out at 60 to 110° and in a mixture of water and an organic solvent or in water containing 0.5 to 3.0 mol of acid per l.

6. Process according to Claim 5, characterised in that the hydrolysis is carried out in water.

## Revendications

1. Procédé pour la préparation des composés de formule : 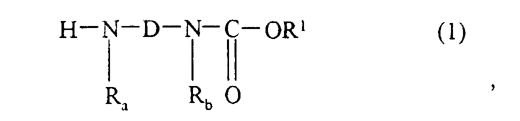 dans laquelle
Rₐ, R_{b} représentent chacun, indépendamment l'un de l'autre, l'hydrogène ou un groupe alkyle éventuellement substitué,
R¹ représente un groupe alkyle, aralkyle, aryle ou cycloalkyle éventuellement substitué et
D représente un radical aromatique divalent,
à l'exception des composés pour lesquels D = phényle à substituant OH et à l'exception des composés, décrits dans le DE-A-40 27 970, qui répondent à la formule (I) : 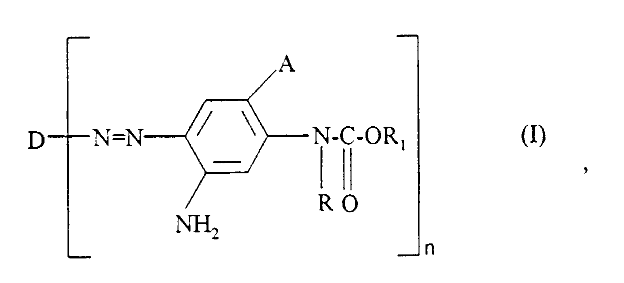 dans laquelle
D représente le radical d'un composant diazotable,
A représente l'hydrogène, un groupe alkyle, alcoxy, un halogène, un groupe carboxy ou sulfo,
R représente l'hydrogène ou un groupe alkyle,
R₁ représente un groupe alkyle, aralkyle, aryle ou cycloalkyle et n est égal à 1 ou 2,
caractérisé en ce que l'on saponifie en milieu acide des composés de formule (2) : 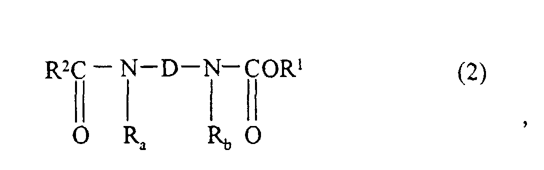 dans laquelle
Rₐ, R_{b}, R¹ et D ont les significations indiquées ci-dessus, et
R² représente l'hydrogène, un groupe alkyle, alcényle, aralkyle, phényle ou carboxy.

2. Procédé selon la revendication 1, pour lequel, dans la formule (I)
D représente un radical éventuellement substitué des séries benzénique, naphtalénique, diphénylique, stilbénique, benzèneazobenzénique, benzèneazonaphtalénique, naphtalèneazonaphtalénique ou hétérocyclique,
Rₐ, R_{b} représentent chacun l'hydrogène ou un groupe alkyle en C₁-C₄ éventuellement substitué,
R¹ représente un groupe alkylc en C₁-C₄ éventuellement substitué, un groupe phényl-alkyle en C₁-C₂ éventuellement substitué dans la partie phényle, un groupe phényle éventuellement substitué ou un groupe cycloalkyle en C₅-C₆ éventuellement substitué.

3. Procédé selon la revendication 1, caractérisé en ce que, dans la formule (I), D est substitué par un groupe alkyle en C₁-C₄, un groupe alcoxy en C₁-C₄, ces groupes alkyle ou alcoxy pouvant eux-mêmes être substitués par OH, -OCH₃, -COOH, -OSO₃H, -SO₃H ; par des groupes -SO₃H, -COOH, des atomes d'halogènes tels que F, Cl ou Br; par NH₂, OH, NO₂, phénylamino dont la partie phényle peut être substituée par CH₃, -OCH₃, -SO₃H ; phénylazo et naphtylazo, ces groupes pouvant eux-mêmes être substitués par -SO₃H.

4. Procédé selon au moins une des revendications qui précèdent, caractérisé en ce que, dans la formule (I), D représente ;

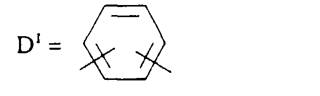

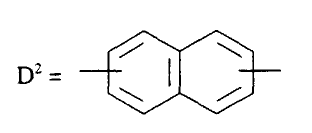

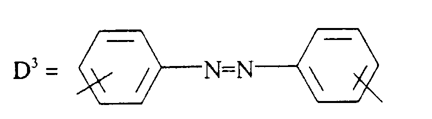

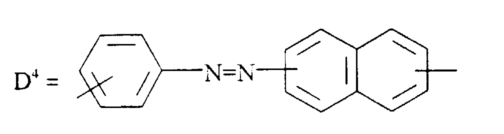

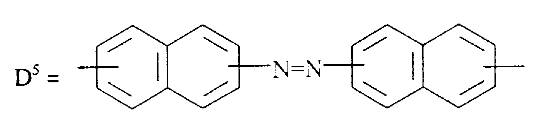

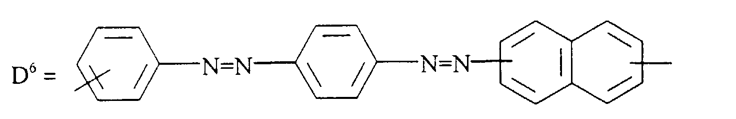

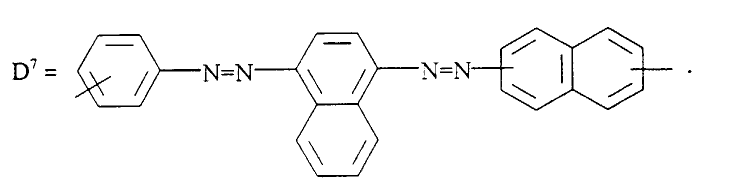

5. Procédé selon la revendication 1, caractérisé en ce que la saponification est réalisée à des températures de 60 à 100° et dans un mélange d'eau et d'un solvant organique ou dans l'eau contenant 0,5 à 3,0 mol d'acide par mol de 1.

6. Procédé selon la revendication 5, caractérisé en ce que la saponification est réalisée dans l'eau.
